# EUROPEAN PATENT SPECIFICATION

(11) **EP 3 463 262 B1**
(45) Date of publication and mention of the grant of the patent: **01.07.2020**
(21) Application number: 17722840.0
(22) Date of filing: 16.05.2017
(51) Int. Cl.: A61K 8/31, A61K 8/36, A61Q 5/02, A61Q 5/12, A61K 8/02

(54) **METHOD OF CONDITIONING HAIR**
HAARKONDITIONIERUNGSVERFAHREN
PROCÉDÉ DE CONDITIONNEMENT CAPILLAIRE

(30) Priority: 27.05.2016 EP 16171817
(43) Date of publication of application: 10.04.2019
(73) Proprietor: Unilever PLC, London, Greater London EC4Y 0DY (GB); Unilever NV, 3013 AL Rotterdam (NL)
(72) Inventor: LI, Ningning, Wirral Merseyside CH63 3JW (GB); MUSCAT, Joseph, Wirral Merseyside CH63 3JW (GB); SAINT-GEORGES, Marine, Pauline, Charlotte, Wirral Merseyside CH63 3JW (GB)
(74) Representative: Chisem, Janet
(86) International application number: PCT/EP2017/061768
(87) International publication number: WO 2017/202654

(56) References cited:
- WO-A1-2007/090554
- WO-A2-2015/082241
- C?CILE A. DREISS: "Wormlike micelles: where do we stand? Recent developments, linear rheology and scattering techniques", SOFT MATTER, vol. 3, no. 8, 1 January 2007 (2007-01-01) , page 956, XP055116849, ISSN: 1744-683X, DOI: 10.1039/b705775j

## Description

### Field of the Invention

This invention relates to a method of conditioning hair. More particularly this invention relates to a method of selectively conditioning damaged hair.

### Background of the Invention

The purpose of bleaching is to eliminate or lighten the natural hair colour by the reaction of an oxidizing agent with the melanin pigment. Examples of oxidizing agents that can be used are hydrogen peroxide, potassium, sodium or ammonium salts of perborate, percarbonate, persulfate and percarbamide, and mixtures thereof.

Bleaches are also used during oxidative dyeing treatments. Oxidative (or "permanent") dye compositions comprise "precursor dyes" which are small molecules capable of diffusing into the hair. These molecules mainly belong to three classes of aromatic compounds: diamines, aminophenols and phenols. They are sufficiently small to diffuse in the hair shaft where, once activated by an oxidizing agent such as hydrogen peroxide, they further react with other precursors to form larger coloured complexes.

Oxidative treatments of hair are very popular with consumers since they provide good results which are relatively unaffected by light, shampooing and perspiration. However the process is not without drawbacks. Repeated oxidative treatments over prolonged periods may damage or weaken hair, making it prone to breakage and reduced lustre.

Silicones are often employed as conditioning materials to improve the feel of hair. However, in the case of hair damaged by treatments such as oxidative treatments, such materials may not provide the same benefit in hair conditioning as for non-oxidatively treated (virgin) hair.

The present invention addresses this problem.

### Summary of the Invention

The invention provides a method of conditioning hair by application of a conditioning shampoo composition, in which the hair is oxidatively-treated hair and in which the conditioning shampoo composition comprises:
(i) an aqueous continuous phase including cleansing surfactant,
(ii) a dispersed phase including emulsified droplets of non-volatile silicone having a mean droplet diameter (D3,2) of 1 micrometre or less, and
(iii) a solubilised oily liquid conditioning agent for skin and/or hair;
in which the oily liquid conditioning agent is solubilised in wormlike micelles in the aqueous continuous phase via the incorporation of at least one inorganic electrolyte and at least one linker molecule which is selected from compounds of general formula R(X)ₙ, in which R is an aryl ring having from 6 to 10 carbon atoms or a mono-, di- or trivalent alkyl or hydroxyalkyl chain having from 3 to 14 carbon atoms; n is 1 to 3 and each X is independently selected from -OH,-COOH and -COO⁻M⁺ groups, where M is an alkali metal, ammonium or alkanolammonium cation;
and in which the level of solubilised oily liquid conditioning agent in the conditioning shampoo composition ranges from 0.45 to 3% by weight based on the total weight of the conditioning shampoo composition.

The invention also provides the use of the conditioning shampoo composition described above for the enhanced conditioning of oxidatively-treated hair compared to virgin hair.

### Detailed Description and Preferred Embodiments

As used herein, the term "oxidatively-treated hair" means hair which has been subjected to any treatment comprising at least one step of contacting the hair with at least one oxidizing composition. Examples of oxidative treatments for human hair are bleaching, dyeing or perming.

As used herein, the term "oxidizing composition" means a composition comprising at least one oxidizing agent suitable for use on hair, such as hydrogen peroxide, potassium, sodium or ammonium salts of perborate, percarbonate, persulfate and percarbamide, and mixtures thereof. Examples of such compositions are oxidative dye compositions and bleaching compositions.

Preferably the oxidatively-treated hair is bleached hair.

As used herein, the term "aqueous continuous phase" is meant a continuous phase which has water as its basis.

Suitably, the composition for use in the invention will comprise from about 50 to about 90%, preferably from about 55 to about 85%, more preferably from about 60 to about 85%, most preferably from about 65 to about 83% water (by weight based on the total weight of the composition).

The cleansing surfactant may suitably be selected from one or more anionic surfactants.

Typical anionic surfactants for use as cleansing surfactants in the invention include those surface active agents which contain an organic hydrophobic group with from 8 to 14 carbon atoms, preferably from 10 to 14 carbon atoms in their molecular structure; and at least one water-solubilising group which is preferably selected from sulphate, sulphonate, sarcosinate and isethionate.

Specific examples of such anionic surfactants include ammonium lauryl sulphate, ammonium laureth sulphate, trimethylamine lauryl sulphate, trimethylamine laureth sulphate, triethanolamine lauryl sulphate, trimethylethanolamine laureth sulphate, monoethanolamine lauryl sulphate, monoethanolamine laureth sulphate, diethanolamine lauryl sulphate, diethanolamine laureth sulphate, lauric monoglyceride sodium sulphate, sodium lauryl sulphate, sodium laureth sulphate, potassium lauryl sulphate, potassium laureth sulphate, sodium lauryl sarcosinate, sodium lauroyl sarcosinate, lauryl sarcosine, ammonium cocoyl sulphate, ammonium lauroyl sulphate, sodium cocoyl sulphate, sodium lauryl sulphate, potassium cocoyl sulphate, potassium lauryl sulphate, monoethanolamine cocoyl sulphate, monoethanolamine lauryl sulphate, sodium tridecyl benzene sulphonate, sodium dodecyl benzene sulphonate, sodium cocoyl isethionate and mixtures thereof.

A preferred class of anionic surfactants for use as cleansing surfactants in the invention are alkyl ether sulphates of general formula:

R-O-(CH₂CH₂-O)ₙ-SO₃⁻M⁺

in which R is a straight or branched chain alkyl group having 10 to 14 carbon atoms, n is a number that represents the average degree of ethoxylation and ranges from 1 to 5, preferably from 2 to 3.5, and M is a alkali metal, ammonium or alkanolammonium cation, preferably sodium, potassium, monoethanolammonium or triethanolammonium, or a mixture thereof.

Specific examples of such preferred anionic surfactants include the sodium, potassium, ammonium or ethanolamine salts of C₁₀ to C₁₂ alkyl sulphates and C₁₀ to C₁₂ alkyl ether sulphates (for example sodium lauryl ether sulphate),

Mixtures of any of the above described materials may also be used.

In a typical composition for use in the invention the level of cleansing surfactant will generally range from 5 to 26% (by weight based on the total weight of the composition).

For the purposes of the present invention, the term "oily liquid" means an oil that is capable of flowing under its own weight under ambient conditions (1 atmosphere, 25°C). The term "oil" means a non-aqueous compound which is immiscible with water (distilled or equivalent) at a concentration of 0.1wt%, at 25°C.

Oily liquid conditioning agents (iii) suitable for use in the invention will generally have a kinematic viscosity at 40°C of 1000 cS (mm².s⁻¹) or less, preferably 500 cS (mm².s⁻¹) or less, more preferably 50 cS (mm².s⁻¹) or less, and most preferably 10 cS (mm².s⁻¹) or less, such as from 0.5 to 10 cS (mm².s⁻¹).

Suitable oily liquid conditioning agents (iii) for use in the invention may generally be selected from cosmetically acceptable oils such as silicone oils, hydrocarbon-based oils and mixtures thereof.

For the purposes of the present invention, the term "silicone oil" means an oil which contains at least one silicon atom, and more particularly at least one Si-O group. The term "hydrocarbon-based oil" means an oil formed from carbon and hydrogen atoms, and optionally oxygen and nitrogen atoms, and not containing any silicon or fluorine atoms. It may contain alcohol, ester, ether, carboxylic acid, amine and/or amide groups. These oils may be of plant, mineral or synthetic origin.

Examples of suitable silicone oils for use in the invention include linear or cyclic silicone oils having a kinematic viscosity of from about 0.65 to about 50, preferably from about 1.5 to about 5 cS (mm².s⁻¹) at 25° C. Example of such materials include linear or cyclic polydimethylsiloxanes having from 2 to 7 siloxane units, such as octamethylcyclotetrasiloxane, decamethylcyclopentasiloxane, dodecamethylcyclohexasiloxane, octamethyltrisiloxane, hexamethyldisiloxane, decamethyltetrasiloxane, dodecamethylpentasiloxane and their mixtures. Preferred are linear polydimethylsiloxanes having from 3 to 5 siloxane units and their mixtures. Such materials are commercially available for example as Dow Corning ® 200 series fluids.

Preferred oily liquid conditioning agents (iii) for use in the invention are generally selected from hydrocarbon-based oils.

Examples of such materials include oily liquid hydrocarbons such as C₄-C₅₀ straight or branched chain, saturated or unsaturated aliphatic or cycloaliphatic hydrocarbons and mixtures thereof. Straight chain hydrocarbons will preferably contain from about 12 to about 30 carbon atoms. Branched chain hydrocarbons can and typically may contain higher numbers of carbon atoms. Also suitable are polymeric hydrocarbons, such as polymers of C₂-₆ alkenyl monomers (e.g. polyisobutene, polybutene) and poly α-olefin oils derived from 1-alkene monomers having from about 6 to about 16 carbons, preferably from about 6 to about 12 carbon atoms (e.g. polymers derived from 1-octene, 1-decene, 1-dodecene, 1- tetradecene, 1-hexadecene, and mixtures thereof). Polymeric hydrocarbons for use in the invention can be straight or branched chain polymers, and may be hydrogenated. The number average molecular weight of such polymeric materials can vary widely, but will typically range from about 200 up to about 3000.

Preferred oily liquid hydrocarbons for use in the invention include mineral oils. The term "mineral oil" in the context of this invention generally denotes an oily liquid mixture of saturated hydrocarbons with boiling-points greater than 200°C, and which is obtained from petroleum (i.e. a mineral source). Mineral oil saturated hydrocarbons include straight chain (paraffinic), branched chain (isoparaffinic) and cyclic (naphthenic) structures, and molecules containing all three configurations, with the number of carbon atoms per hydrocarbon molecule generally ranging from about C₁₅ to about C₅₀. Mineral oils suitable for use in the invention are typically obtained from petroleum through various refining steps (e.g. distillation, extraction and/or crystallisation) and subsequent purification (e.g. acid treatment and/or catalytic hydrotreatment).

Mineral oils may also be characterised in terms of their viscosity. "Light" mineral oils will generally have a kinematic viscosity of about 34 cS (mm².s⁻¹) or less at 40°C and "heavy" mineral oils will generally have a kinematic viscosity ranging from about 35 cS (mm².s⁻¹) up to about 240 cS (mm².s⁻¹) at 40°C.

Light mineral oils (as defined above) are preferred for use in the invention. More preferably such light mineral oils have a kinematic viscosity of about 10 cS (mm².s⁻¹) or less at 40°C. Most preferably the kinematic viscosity ranges from about 3 to about 5 cS (mm².s⁻¹) at 40°C. Materials of this type are commercially available from Sonneborn Inc. under the brand name Lytol®.

Other suitable hydrocarbon-based oils for use in the invention include oily liquid esters. Oily liquid esters for use in the invention are generally characterised by having at least 10 carbon atoms, and may be either straight-chained or branched. The esters may have hydrocarbyl chains derived from fatty acids or alcohols (e.g. monoesters, polyhydric alcohol esters, and di- and tri-carboxylic acid esters). The hydrocarbyl radicals may include or have covalently bonded thereto other compatible functionalities, such as amides and alkoxy moieties, such as ethoxy or ether linkages.

Examples of oily liquid esters for use in the invention include:
aliphatic monohydric alcohol esters such as C₅-C₂₂ straight or branched-chain, saturated or unsaturated alkyl esters of C₁-C₁₈ straight or branched-chain, saturated or unsaturated alkyl alcohols (provided that the total number of carbon atoms in the ester is at least 10), such as isostearyl palmitate, isononyl isononanoate, myristyl propionate, isopropyl isostearate, isopropyl myristate, isopropyl palmitate, ethylhexyl palmitate, cetyl acetate, cetyl propionate, cetyl stearate, isodecyl neopentanoate, cetyl octanoate, isocetyl stearate , ethylhexyl stearate and mixtures thereof;
aliphatic polyhydric alcohol esters such as C₅-C₂₂ straight or branched-chain, saturated or unsaturated alkyl esters of C₃-C₃₀ straight or branched-chain, saturated or unsaturated polyols (provided that the total number of carbon atoms in the ester is at least 10), such as propylene glycol dipelargonate, pentaerythrityl tetraoctanoate, trimethylolpropane tricaprylate/tricaprate, trioctanoin, pentaerythrityl tetrapelargonate, sorbitan trioleate, caprylic/capric triglyceride, neopentyl alcohol tetraoctanoate, and mixtures thereof;
aliphatic polycarboxylic acid polyesters such as C₅-C₂₂ straight or branched-chain, saturated or unsaturated alkyl diesters of C₂-C₁₀ straight or branched-chain, saturated or unsaturated dicarboxylic acids (provided that the total number of carbon atoms in the ester is at least 10), such as diisopropyl adipate, dioctyl sebacate, dioctyl succinate, dioctyl maleate, diisostearyl adipate, diethyl sebacate, diisostearyl fumarate, dioctyl adipate and mixtures thereof; and/or C₅-C₂₂ straight or branched-chain, saturated or unsaturated alkyl triesters of C₆-C₁₀ straight or branched-chain, saturated or unsaturated tricarboxylic acids (provided that the total number of carbon atoms in the ester is at least 10), such as trioctyldodecyl citrate, triisostearyl citrate, triisopropyl citrate and mixtures thereof; and
aliphatic esters of aromatic acids such as C₁₂-C₁₅ branched or unsaturated alkyl esters of benzoic acid.

Preferred oily liquid esters for use in the invention may be selected from the aliphatic monohydric and/or polyhydric alcohol esters which are described in more detail above.

Mixtures of any of the above-described materials may also be used.

The level of oily liquid conditioning agent (iii) in compositions for use in the invention depends on the particular material (s) used, but generally ranges from about 0.5 to about 3% by weight based on the total weight of the composition.

In a preferred composition for use in the invention the oily liquid conditioning agent (iii) is selected from oily liquid hydrocarbons, oily liquid esters and mixtures thereof, at a level ranging from about 0.45 to about 2%, more preferably from about 0.5 to about 1.5% (by weight based on the total weight of the composition).

In a particularly preferred composition for use in the invention the oily liquid conditioning agent (iii) is light mineral oil (as defined above), at a level ranging from about 0.5 to about 1.5% (by weight based on the total weight of the composition).

In the composition for use in the invention, the oily liquid conditioning agent (iii) is solubilised in wormlike micelles in the aqueous continuous phase (i). Typically the solubilised oily liquid conditioning agent (iii) forms a microemulsion which is stable to phase separation.

"Wormlike micelles" in the context of this invention are elongated and flexible aggregates formed by the self-assembly of surfactant molecules in water. Above a threshold concentration, wormlike micelles entangle into a transient network, reminiscent of polymer solutions, and display viscoelastic properties. However, unlike a covalently bonded polymer backbone, the micelles are in a state of thermodynamic equilibrium with the solvent and are perpetually broken and reformed under Brownian fluctuations. This leads to a broad and dynamic distribution of micelle lengths which can change under an imposed shear or extensional flow.
Wormlike micelles can be fully described by a number of structural parameters, which cover a broad range of length-scales. The overall length of the micelles is referred to as the contour length L and varies between a few (e.g. about 1 to 10) nanometers up to a few (e.g. about 1 or 2) microns. Cryo-TEM provides a direct visualization of the micelles and can be used to estimate the contour length, while light and neutron scattering give a more accurate determination. Radii of wormlike micelles are typically a few (e.g. about 1 to 10) nm. Another key structural parameter in the description of wormlike micelles is the persistence length *I*ₚ, the length over which the micelles are considered rigid. Although wormlike micelles can be extremely flexible and micrometres long, their large cross-section implies that on smaller length-scales (of order *I*ₚ) they act as rigid rods. Techniques such as rheology, light and neutron scattering and flow birefringence have been employed to estimate *I*ₚ, as well as simulations. Experimentally, persistence lengths from about 10 to about 40 nm have been reported in neutral systems. For charged wormlike micelles, the persistence length varies significantly with surfactant structure, counter-ion and salt concentration, but is typically a few tens of nanometers (e.g about 30 to about 100 nm).

The oily liquid conditioning agent (iii) is solubilised in wormlike micelles in the aqueous continuous phase via the incorporation of at least one inorganic electrolyte and at least one linker molecule of general formula R(X)ₙ as defined above.
"Linker molecules" in the context of this invention are chemical additives used in surfactant systems that enhance the surfactant-oil or surfactant-water interactions. Lipophilic linkers segregate near the oil side of the interface close to the tails of the surfactants. The presence of the lipophilic linker extends the impact of the surfactant deeper into the oil phase and may promote additional orientation of the oil molecules. Hydrophilic linkers are surfactant-like molecules that coadsorb with the surfactant at the oil/water interface, but have a minimal interaction with the oil molecules. The adsorption of the hydrophilic linker at the oil/water interface increases the total interfacial area.

R in the general formula R(X)ₙ above is preferably a phenyl ring or a mono-, di- or trivalent linear alkyl or hydroxyalkyl chain having from 3 to 12 carbon atoms.

Preferred linker molecules for use in the invention include:
aromatic carboxylic acids of formula R(X)ₙ as defined above, in which R is a phenyl ring; n is 1 or 2 and each X is independently selected from -COOH and -COO⁻M⁺ groups, where M is as defined above, preferably sodium or potassium;
linear aliphatic mono- , di- or tricarboxylic acids of formula R(X)ₙ as defined above, in which R is a mono-, di- or trivalent, linear, alkyl or hydroxyalkyl chain having from 3 to 12, preferably from 6 to 10 carbon atoms, and each X is independently selected from -COOH and -COO⁻M⁺ groups, where M is as defined above, preferably sodium or potassium, and
linear aliphatic diols of formula R(X)ₙ as defined above, in which R is a divalent linear alkyl chain having from 3 to 12 carbon atoms.

Examples of preferred linker molecules for use in the invention include benzoic acid, citric acid, phthalic acid, caprylic acid, lauric acid, azelaic acid (and/or their sodium or potassium salts) and 1,12-dodecanediol.

Mixtures of any of the above described materials may also be suitable.
The level of linker molecule (as defined above) in compositions for use in the invention preferably ranges from about 0.01 to about 1%, more preferably from about 0.02 to about 0.5%, most preferably from about 0.05 to about 0.15% by weight based on the total weight of the composition.

The weight ratio of solubilised oily liquid conditioning agent (iii) to linker molecule (as defined above) in compositions for use in the invention generally ranges from about 15:1 to about 1:1, preferably from about 12:1 to about 6:1, more preferably from about 10:1 to about 8:1.

A particularly preferred composition for use in the invention comprises caprylic acid as the linker molecule, in combination with light mineral oil as the solubilised oily liquid conditioning agent (iii), in the amounts and ratios given above.
The composition for use in the invention includes at least one inorganic electrolyte. The inorganic electrolyte is used to assist in the solubilisation of the oily liquid conditioning agent (iii) and to provide viscosity to the composition.

The viscosity of the composition for use in the invention suitably ranges from 3,000 to 10,000 mPa.s, preferably from 4,000 to 9,000 mPa.s when measured using a Brookfield V2 viscometer (spindle RTV5, 1 minute, 20rpm) at 30°C.

Suitable inorganic electrolytes include metal chlorides (such as sodium chloride, potassium chloride, calcium chloride, magnesium chloride, zinc chloride, ferric chloride and aluminium chloride) and metal sulphates (such as sodium sulphate and magnesium sulphate).

Examples of preferred inorganic electrolytes for use in the invention include sodium chloride, potassium chloride, magnesium sulphate and mixtures thereof.

Mixtures of any of the above described materials may also be suitable.

The level of inorganic electrolyte in compositions for use in the invention depends on the particular oily liquid conditioning agent (iii) used, but generally ranges from about 1 to about 25%, preferably from about 1.5 to about 20% (by total weight inorganic electrolyte based on the total weight of the composition).

A particularly preferred composition for use in the invention comprises sodium chloride as the inorganic electrolyte, in the amounts given above.

The composition for use in the invention comprises a dispersed phase (ii) including emulsified droplets of non-volatile silicone having a mean droplet diameter (D3,2) of 1 micrometre or less. Preferably the mean droplet diameter (D3,2) is 1 micrometre or less, more preferably 0.5 micrometre or less, and most preferably 0.25 micrometre or less.

A suitable method for measuring the mean droplet diameter (D3,2) is by laser light scattering using an instrument such as a Malvern Mastersizer.

The term "non-volatile silicone" in the context of this invention means a silicone with a vapour pressure of less than 1000 Pa at 25°C.

Suitable silicones for use in the invention include polydiorganosiloxanes, in particular polydimethylsiloxanes (dimethicones), polydimethyl siloxanes having hydroxyl end groups (dimethiconols), and amino-functional polydimethylsiloxanes (amodimethicones).

Suitable silicones preferably have a molecular weight of greater than 100,000 and more preferably a molecular weight of greater than 250,000.

All molecular weights as used herein are weight average molecular weights, unless otherwise specified.

Suitable silicones preferably have a kinematic viscosity of greater than 50,000 cS
(mm².s⁻¹) and more preferably a kinematic viscosity of greater than 500,000 cS (mm².s⁻¹). Silicone kinematic viscosities in the context of this invention are measured at 25°C and can be measured by means of a glass capillary viscometer as set out further in Dow Corning Corporate Test Method CTM004 July 20, 1970.

Suitable silicones for use in the invention are available as pre-formed silicone emulsions from suppliers such as Dow Corning and GE Silicones. The use of such pre-formed silicone emulsions is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer. Pre-formed silicone emulsions having a mean droplet diameter (D3,2) of less than 0.15 micrometers are generally termed microemulsions.
Examples of suitable pre-formed silicone emulsions include emulsions DC2-1766, DC2-1784, DC-1785, DC-1786, DC-1788, DC-1310, DC-7123 and microemulsions DC2-1865 and DC2-1870, all available from Dow Corning. These are all emulsions/microemulsions of dimethiconol. Also suitable are amodimethicone emulsions such as DC939 (from Dow Corning) and SME253 (from GE Silicones).

Mixtures of any of the above described silicone emulsions may also be used.

The amount of emulsified, non-volatile silicone in compositions for use in the invention may suitably range from 0.05 to 10%, preferably from 0.2 to 8% (by total weight silicone based on the total weight of the composition).

The composition for use in the invention preferably includes one or more cationic polymers. Such polymers may enhance the delivery of conditioning agents and thereby improve the conditioning benefits obtained.

Cationic polymers typically contain cationic nitrogen-containing groups such as quaternary ammonium or protonated amino groups. The cationic protonated amines can be primary, secondary, or tertiary amines (preferably secondary or tertiary). The average molecular weight of the cationic polymer is preferably from 5,000 to 10 million. The cationic polymer preferably has a cationic charge density of from 0.2 meq/gm to 7 meq/gm.

The term "cationic charge density" in the context of this invention refers to the ratio of the number of positive charges on a monomeric unit of which a polymer is comprised to the molecular weight of the monomeric unit. The charge density multiplied by the polymer molecular weight determines the number of positively charged sites on a given polymer chain.

The cationic nitrogen-containing moiety of the cationic polymer is generally present as a substituent on all, or more typically on some, of the repeat units thereof. The cationic polymer may be a homo-polymer or co-polymer of quaternary ammonium or cationic amine-substituted repeat units, optionally in combination with non-cationic repeat units. Particularly suitable cationic polymers for use in the invention include cationic polysaccharide polymers, such as cationic cellulose derivatives, cationic starch derivatives, and cationic guar gum derivatives.

A particularly suitable type of cationic polysaccharide polymer that can be used is a cationic guar gum derivative, such as guar hydroxypropyltrimethylammonium chloride. (commercially available from Rhodia® in their JAGUAR® trademark series). Examples of such materials are JAGUAR ® C13S, JAGUAR ® C14, JAGUAR® C15 and JAGUAR ® C17.

Mixtures of any of the above described cationic polymers may also be used.

When included, the total level of cationic polymer in the composition for use in the invention is preferably from 0.05% to 2% and more preferably from 0.1 to 0.5% by weight based on the total weight of the composition.

The composition for use in the invention preferably includes one or more amphoteric surfactants. Suitable amphoteric surfactants are betaines, such as those having the general formula R(CH₃)₂N⁺CH₂COO⁻, where R is an alkyl or alkylamidoalkyl group, the alkyl group preferably having 10 to 16 carbon atoms. Particularly suitable betaines are oleyl betaine, caprylamidopropyl betaine, lauramidopropyl betaine, isostearylamidopropyl betaine, and cocoamidopropyl betaine.

When included, the total level of amphoteric surfactant is generally from 0.1% to 20%, preferably from 1% to 10%, more preferably from 1% to 5% by weight based on the total weight of the composition.

The composition for use in the invention preferably includes one or more suspending agents. Suitable suspending agents include polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives.

Mixtures of any of the above suspending agents may be used. Preferred is a mixture of cross-linked polymer of acrylic acid and crystalline long chain acyl derivative.

When included, the total level of suspending agent is generally 0.1 to 10%, preferably from 0.5 to 6%, more preferably from 0.9 to 4% by weight based on the total weight of the composition.

A composition for use in the invention may contain further optional ingredients to enhance performance and/or consumer acceptability. Examples of such ingredients include fragrance, dyes and pigments, pH adjusting agents and preservatives or antimicrobials. Each of these ingredients will be present in an amount effective to accomplish its purpose. Generally these optional ingredients are included individually at a level of up to 5% by weight based on the total weight of the composition.

Typically the composition for use in the invention is topically applied to the hair and then massaged into the hair and scalp. The composition is then rinsed off the hair and scalp with water prior to drying the hair.
The invention will be further illustrated by the following, non-limiting Examples, in which all percentages quoted are by weight based on total weight unless otherwise stated.

### EXAMPLES

Hair cleansing shampoo formulations were prepared, having ingredients as shown in Table 1. Examples 1 to 4 represent formulations according to the invention.

**Table 1**

| **Ingredient** | **Control (%w/w)** | **Example 1 (%w/w)** | **Example 2 (%w/w)** | **Example 3 (%w/w)** | **Example 4 (%w/w)** |
|---|---|---|---|---|---|
| Sodium laureth sulphate (1EO) | 11.6 | 11.6 | 11.6 | 11.6 | 11.6 |
| Cocamidopropyl betaine | 1.5 | 1.5 | 1.5 | 1.5 | 1.5 |
| Guar hydroxypropyltrimonium chloride | 0.2 | 0.2 | 0.2 | 0.2 | 0.2 |
| Dimethiconol* | 1 | 1 | 1 | 1 | 1 |
| Carbo mer | 0.4 | 0.4 | 0.4 | 0.4 | 0.4 |
| Ethylene glycol distearate | 0.6 | 0.6 | 0.6 | 0.6 | 0.6 |
| Mica | 0.2 | 0.2 | 0.2 | 0.2 | 0.2 |
| Disodium EDTA | 0.1 | 0.1 | 0.1 | 0.1 | 0.1 |
| Zinc pyrithione | 1 | 1 | 1 | 1 | 1 |
| Sodium hydroxide | 0.2 | 0.2 | 0.3 | 0.3 | 0.3 |
| Sodium chloride | 0.5 | 2 | 3 | 3.6 | 4.2 |
| Caprylic acid | 0 | 0.05 | 0.1 | 0.15 | 0.2 |
| Lytol® mineral oil | 0 | 0.45 | 0.9 | 1.35 | 1.8 |
| Water,perfume,preservatives | to 100% | to 100% | to 100% | to 100% | to 100% |

| | | | | | |
|---|---|---|---|---|---|
| ** Emulsion of dimethiconol with anionic emulsifier, average particle size* < *1 micron (ex Dow Corning)* | | | | | |

### Measurement of silicone deposition

The formulations described in Table 1 were assessed for their silicone deposition onto oxidatively-treated hair using the following protocol:
0.25g test formulation is applied to a wet 2.5g/6" switch of double bleached European hair. The test formulation is massaged on the switch for 30 seconds followed by rinsing with warm water for 30 seconds. This treatment is repeated twice. Silicone deposition is measured by X-ray Fluorescence (XRF). Five replicas were produced for each test formulation. The average of measured silicone deposition is shown in Table 2.

**Table 2**

| **Test formulation** | **Control** | **Example 1** | **Example 2** | **Example 3** | **Example 4** |
|---|---|---|---|---|---|
| **Mean deposition/ppm** | **352** | **472** | **449** | **454** | **386** |
| Standard deviation | 48 | 77 | 89 | 93 | 110 |

The results show that, Examples 1 to 4 deposit significantly more silicone than the control. This improved silicone deposition was confirmed as statistically significant by t-test.

The improvement in silicone deposition was also observed to be superior to the results obtained when the tests were repeated using virgin dark brown European (DBE) hair switches in place of the double bleached switches.

## Claims

1. A method of conditioning hair by application of a conditioning shampoo composition, in which the hair is oxidatively-treated hair and in which the conditioning shampoo composition comprises:
(i) an aqueous continuous phase including cleansing surfactant,
(ii) a dispersed phase including emulsified droplets of non-volatile silicone having a mean droplet diameter (D3,2) of 1 micrometre or less, and
(iii) a solubilised oily liquid conditioning agent for skin and/or hair;
in which the oily liquid conditioning agent is solubilised in wormlike micelles in the aqueous continuous phase via the incorporation of at least one inorganic electrolyte and at least one linker molecule which is selected from compounds of general formula R(X)ₙ, in which R is an aryl ring having from 6 to 10 carbon atoms or a mono-, di- or trivalent alkyl or hydroxyalkyl chain having from 3 to 14 carbon atoms; n is 1 to 3 and each X is independently selected from -OH, -COOH and -COO⁻M⁺ groups, where M is an alkali metal, ammonium or alkanolammonium cation;
and in which the level of solubilised oily liquid conditioning agent in the conditioning shampoo composition ranges from 0.45 to 3% by weight based on the total weight of the conditioning shampoo composition.

2. A method according to claim 1, in which the oxidatively-treated hair is bleached hair.

3. A method according to claim 1 or claim 2, in which the oily liquid conditioning agent is light mineral oil having a kinematic viscosity of 3 to 5 cS (mm².s⁻¹) at 40°C.

4. A method according to claim 3, in which the level of the light mineral oil ranges from 0.5 to 1.5% by weight based on the total weight of the conditioning shampoo composition.

5. A method according to any preceding claim, in which R is a phenyl ring or a mono-, di- or trivalent linear alkyl or hydroxyalkyl chain having from 3 to 12 carbon atoms.

6. A method according to claim 5, in which the linker molecule is caprylic acid.

7. A method according to claim 6, in which the level of the caprylic acid ranges from 0.05 to 0.15% by weight based on the total weight of the composition.

8. Use of the conditioning shampoo composition as defined in any one of Claims 1 to 7 for the enhanced conditioning of oxidatively-treated hair compared to virgin hair.

## Patentansprüche

1. Verfahren zur Haarkonditionierung durch Auftragen einer Konditionierungsshampoozusammensetzung, in welcher das Haar oxidativ behandeltes Haar ist und in welcher die Konditionierungsshampoozusammensetzung umfasst:
(i) eine wässrige kontinuierliche Phase, die ein Reinigungstensid enthält,
(ii) eine dispergierte Phase, die emulgierte Tröpfchen von nicht-flüchtigem Silicon eines mittleren Tröpfchendurchmessers (D3,2) von 1 Mikrometer oder weniger enthält, und
(iii) ein solubilisiertes öliges flüssiges Konditionierungsmittel für Haut und/oder Haar;
in welcher das ölige flüssige Konditionierungsmittel in wurmähnlichen Mizellen in der wässrigen kontinuierlichen Phase über die Einverleibung mindestens eines anorganischen Elektrolyten und mindestens eines Linker-Moleküls, welches unter Verbindungen der allgemeinen Formel R(X)ₙ ausgewählt ist, solubilisiert ist, in welcher R einen Arylring mit 6 bis 10 Kohlenstoffatomen oder eine ein-, zwei- oder dreiwertige Alkyl- oder Hydroxyalkylkette mit 3 bis 14 Kohlenstoffatomen darstellt; n 1 bis 3 ist und jedes X unabhängig unter -OH, -COOH und -COO⁻M⁺-Gruppen, wobei M ein Alkalimetall-, Ammonium- oder Alkanolammoniumkation ist, ausgewählt ist;
und in welchem der Anteil des solubilisierten öligen flüssigen Konditionierungsmittels in der Konditionierungsshampoo-Zusammensetzung in dem Bereich von 0,45 bis 3 Gewichts-%, bezogen auf das Gesamtgewicht der Konditionierungsshampoo-Zusammensetzung, liegt.

2. Verfahren nach Anspruch 1, in welchem das oxidativ behandelte Haar gebleichtes Haar ist.

3. Verfahren nach Anspruch 1 oder Anspruch 2, in welchem das ölige flüssige Konditionierungsmittel leichtes Mineralöl einer kinematischen Viskosität von 3 bis 5 cS (mm².s⁻¹) bei 40°C ist.

4. Verfahren nach Anspruch 3, in welchem der Anteil des leichten Mineralöls in dem Bereich von 0,5 bis 1,5 Gewichts-%, bezogen auf das Gesamtgewicht der Konditionierungsshampoo-Zusammensetzung, ist.

5. Verfahren nach irgendeinem vorhergehenden Anspruch, in welchem R ein Phenylring oder eine ein-, zwei- oder dreiwertige lineare Alkyl- oder Hydroxyalkylkette mit 3 bis 12 Kohlenstoffatomen ist.

6. Verfahren nach Anspruch 5, in welchem das Linkermolekül Caprylsäure ist.

7. Verfahren nach Anspruch 6, in welchem der Anteil der Caprylsäure in dem Bereich von 0,05 bis 0,15 Gewichts-%, bezogen auf das Gesamtgewicht der Zusammensetzung, liegt.

8. Verwendung der Konditionierungsshampoo-Zusammensetzung, wie in irgendeinem der Ansprüche 1 bis 7 definiert, zum verbesserten Konditionieren von oxidativ behandeltem Haar, verglichen mit unberührtem Haar.

## Revendications

1. Procédé de conditionnement des cheveux par application d'une composition de shampoing conditionnant, dans lequel les cheveux sont des cheveux traités par oxydation et dans lequel la composition de shampoing conditionnant comprend :
(i) une phase continue aqueuse incluant un tensioactif nettoyant,
(ii) une phase dispersée incluant des gouttelettes émulsionnées de silicone non-volatil ayant un diamètre moyen de gouttelette (D3,2) de 1 micromètre ou inférieur, et
(iii) un agent conditionnant liquide huileux solubilisé pour la peau et/ou les cheveux ;
dans lequel l'agent conditionnant liquide huileux est solubilisé dans des micelles semblables à des vers dans la phase continue aqueuse via l'incorporation d'au moins un électrolyte inorganique et d'au moins une molécule de liaison qui est choisie parmi des composés de formule générale R(X)ₙ, dans laquelle R est un noyau aryle ayant de 6 à 10 atomes de carbone ou une chaîne alkyle ou hydroxyalkyle mono-, di- ou trivalente ayant de 3 à 14 atomes de carbone ; n est égal à de 1 à 3 et chaque X est indépendamment choisi parmi les groupes -OH, -COOH et -COO⁻M⁺, où M est un métal alcalin, un cation ammonium ou alcanolammonium ;
et dans lequel la teneur d'agent conditionnant liquide huileux solubilisé dans la composition de shampoing conditionnant est de 0,45 à 3 % en masse rapportée à la masse totale de la composition de shampoing conditionnant.

2. Procédé selon la revendication 1, dans lequel les cheveux traités par oxydation sont des cheveux décolorés.

3. Procédé selon la revendication 1 ou la revendication 2, dans lequel l'agent conditionnant liquide huileux est une huile minérale légère ayant une viscosité cinématique de 3 à 5 cS (mm².s⁻¹) à 40°C.

4. Procédé selon la revendication 3, dans lequel la teneur de l'huile minérale légère est de 0,5 à 1,5 % en masse rapportée à la masse totale de la composition de shampoing conditionnant.

5. Procédé selon l'une quelconque des revendications précédentes, dans lequel R est un noyau phényle ou une chaîne alkyle ou hydroxyalkyle mono-, di- ou trivalente linéaire ayant de 3 à 12 atomes de carbone.

6. Procédé selon la revendication 5, dans lequel la molécule de liaison est l'acide caprylique.

7. Procédé selon la revendication 6, dans lequel la teneur de l'acide caprylique est de 0,05 à 0,15 % en masse rapportée à la masse totale de la composition.

8. Utilisation de la composition de shampoing conditionnant selon l'une quelconque des revendications 1 à 7 pour le conditionnement amélioré de cheveux traités par oxydation en comparaison avec des cheveux vierges.
